# EUROPEAN PATENT APPLICATION

(11) **EP 3 184 041 A1**
(43) Date of publication of application: **28.06.2017**
(21) Application number: 15201846.1
(22) Date of filing: 22.12.2015
(51) Int. Cl.: A61B 5/024, A61B 5/0408

(54) **SYSTEM AND DEVICE FOR MONITORING HEART ACTIVITIES OF A USER OF THE DEVICE**

(71) Applicant: Sony Mobile Communications, Inc., Minato-ku Tokyo 108-0075 (JP)
(72) Inventor: SIOTIS, Georg, 222 21 Lund (SE)
(74) Representative: Neij & Lindberg AB

(57) **Abstract**

An accessory, system and method for monitoring an electrical footprint of the heart of a user is provided, wherein the accessory comprises a housing constructed to be worn around a neck of a user, comprising two or more conductive contact surfaces configured to detect an electrical footprint of the heart of a user.

## Description

### TECHNICAL FIELD

The disclosure pertains to the field of providing health related information associated with a user. In particular it discloses devices, system and methods for monitoring and transmitting the heart activity related information.

### BACKGROUND ART

Known devices and methods of monitoring data and controlling data transfer between portable electronic devices, by wire or wirelessly, are relatively straight-forward for a user of mobile devices and wearables, such as mobile telephones, headphones, headsets, microphones, speakers and/or wristlets. Recent interest in personal health has led to a variety of portable personal health monitoring devices being developed and offered on the market.

There is an existing trend in combining music listening with physical activity or health monitoring, such as step counter etc., i.e. accelerometers are installed in headsets for that purpose.

There also exists different Heart Rate Monitor methods, like the traditional Plethysmographic wrist worn device, i.e. Apple Watch, Everest, Up3 etc., the traditional chest mounted band or to include the ECG or Pyroelectric sensors in the ear pinna.

With increased functionality of health monitoring devices often comes increased development cost, both for the hardware and software. Furthermore, increased hardware also leads to bulkier monitoring devices. Increased hardware and/or more advanced software may also lead to decreased battery time for wireless monitoring devices.

The existing Heart Rate Monitor methods are both rather cumbersome an the plethysmographic sensors are sensible to the colour of the skin colour of the skin.

In summary, there is a need to reduce development cost for monitoring devices, reduce bulkiness and decrease battery consumption to increase their usefulness to users.

### SUMMARY OF THE INVENTION

With the above description in mind, then, an aspect of the present invention is to provide devices and methods for monitoring personal health information, such as the electrical footprint of the heart of a user, by using a wireless communication device, which seek to mitigate, alleviate, or eliminate one or more of the above-identified deficiencies in the art and disadvantages singly or in any combination.

These and further objects are achieved by devices and methods equipped with, and utilizing different kinds of short range wireless solutions, also referred to as Personal Area Network (PAN) solutions. Examples are; Bluetooth^{®} (BT), infrared Data Association (IrDA), ZigBee^{®}, Ultra-WideBand (UWB), etc.

The present disclosure is defined by the appended claims. Various advantageous embodiments of the disclosure are set forth by the appended claims as well as by the following description and the accompanying drawings.

According to some aspects the disclosure provides for a wireless communication accessory. The accessory comprising housing constructed to be worn around the neck of a user, two or more conductive contact surfaces configured to detect an electrical footprint of the heart of a user and an electronic module. The electronic module comprising a communication circuit and a processor configured to process the detected electrical footprint. Further, the communication circuit, processor and the two or more conductive contact surfaces are communicatory connected to each other, the housing is constructed to be kept in contact with the skin of the user by using the gravitation force, G, and the two or more conductive contact surfaces are positioned on said housing such that said conductive contact surfaces are in direct contact with the skin of a user when said housing is positioned around the neck of said user. In one aspect the conductive contact surface is made of any of silver, plated brass or copper.

In one embodiment the housing is constructed to be kept in contact with the skin of the user by using a spring effect of the construction of the housing. The spring effect is especially effectfull in keeping the accessory in place when the user performs som kind of activity, such as dancing or running.

In one embodiment the accessory is provided with four or more conductive contact surfaces and the processor is configured to select which pair of conductive contact surfaces detects the strongest electrical footprint signal of the heart.

In one embodiment the electrical footprint of the heart is an electrocardiogram, ECG, signal and the processor is configured to calculate heart rate beats per minute, bpm, based on the detected electrocardiogram, ECG, signal.

In one embodiment the processor is configured to amplify said detected electrocardiogram, ECG, signal with amplitude of 1 mV, to subtract common mode frequency component and to filter out frequency components above 5 Hz before calculate the heart rate beats per minute, bpm.

In one embodiment the communication circuitry is configured to transmit the detected electrical footprint of the heart of a user to a wireless communication device by using the communication circuitry.

According to some aspects the disclosure provides for a wireless system for monitoring an electrical footprint of the heart of a user. The system comprises a wireless communication device and a wireless communication accessory connected to the wireless communication device. The wireless communication accessory comprises a housing constructed to be worn around the neck of a user, a communication circuit, two or more conductive contact surfaces configured to detect the electrical footprint of the heart of a user and a processor configured to process the detected electrical footprint. The communication circuit, processor and the two or more conductive contact surfaces are communicatory connected to each other. The housing is constructed to be kept in contact with the skin of the user by using the gravitation force, G. The two or more conductive contact surfaces are positioned on the housing such that the conductive contact surfaces are in direct contact with the skin of a user when the housing is positioned around the neck of said user.

In one embodiment, the housing is constructed to be kept in contact with the skin of the user by using a spring effect of the construction of the housing.

In one embodiment the accessory is provided with four or more conductive contact surfaces and the processor is configured to select which pair of conductive contact surfaces detects the strongest electrical footprint signal of the heart.

In one embodiment the processor is configured to amplify said detected electrocardiogram, ECG, signal with amplitude of 1 mV, to subtract common mode frequency component, to filter out frequency components above 5 Hz and to calculate heart rate beats per minute, bpm, based on said detected electrocardiogram, ECG, signal.

According to some aspects the disclosure provides for a method for monitoring an electrical footprint of the heart of a user by using a wireless communication accessory connected to a wireless communication device. The method comprises detecting said electrical footprint of the heart of the user by using two or more conductive contact surfaces positioned on said wireless communication accessory, transmitting said detected footprint to the communication device and processing said transmitted footprint.

In one embodiment the wireless communication accessory is provided with four or more conductive contact surfaces and the detecting comprises selecting a pair of conductive contact surfaces that detects the strongest electrical footprint signal of the heart.

In one embodiment the processing further comprises amplifying the detected electrocardiogram, ECG, signal with amplitude of 1 mV, subtracting common mode frequency component, filtering out frequency components above 5 Hz and calculating heart rate beats per minute, bpm, based on said detected electrocardiogram, ECG, signal.

According to some aspects the disclosure provides for a computer program comprising computer readable code which, when run on an electronic device causes the electronic device to perform the method as described above.

With the above description in mind, aspects of the present disclosure overcome at least some of the disadvantages of known technology as previously described.

### BRIEF DESCRIPTION OF THE DRAWINGS

The foregoing will be apparent from the following more particular description of the example embodiments, as illustrated in the accompanying drawings in which like reference characters refer to the same parts throughout the different views. The drawings are not necessarily to scale, emphasis instead being placed upon illustrating the example embodiments.
Figure 1 illustrates an example of a user wearing an accessory comprising a headset with an associated collar according to the present invention.
Figure 2a-2b illustrates examples of accessories worn arround the neck of a user.

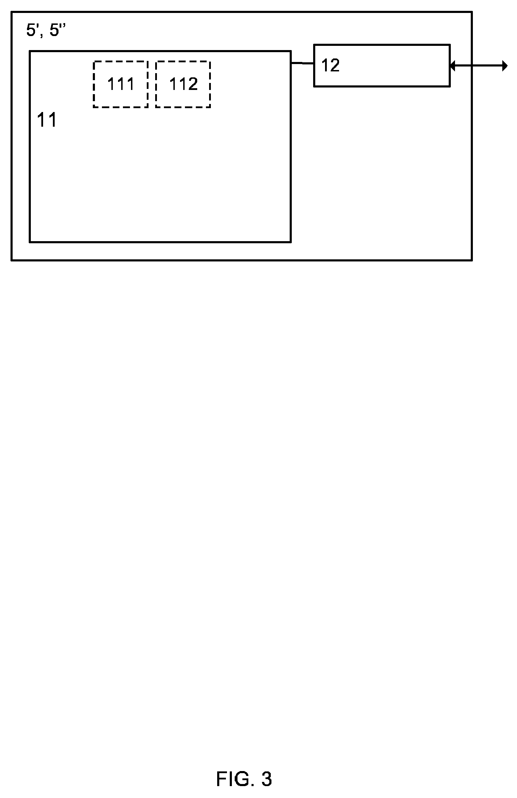

Figure 3 illustrates an electronic module within said collar.
Figure 4 is a flow chart illustrating the proposed method, performed in the collar.
Figure 5 illustrates an example of an electronic device according to the present invention.
Figure 6 is a flow chart illustrating the proposed method, performed in the electronic device according to the present invention.

It should be added that the following description of the aspects/embodiments is for illustration purposes only and should not be interpreted as limiting the disclosure exclusively to these embodiments/aspects.

### DETAILED DESCRIPTION

Aspects of the present disclosure will be described more fully hereinafter with reference to the accompanying drawings. The devices and methods disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the invention. As used herein, the singular forms "a", "an" and "the" are intended to include the plural forms as well, unless the context clearly indicates otherwise.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

A wireless communication accessory with a built-in heart monitoring device is provided, with a couple of conductive surfaces or electrodes provided on a housing of the accessory shaped like a collar or the like worn around the neck of the user.

Embodiments of the present invention will be exemplified using an electronic device such as a mobile communication device such as a mobile phone. However, it should be appreciated that the invention is as such equally applicable to electronic devices which have touch detection capabilities. Examples of such devices may for instance be any type of mobile phone, smartphone, laptop (such as standard, ultra-portables, netbooks, and micro laptops) handheld computers, portable digital assistants, tablet computers, touch pads, gaming devices, accessories to mobile phones, e.g. wearables in the form of headphones/-sets, visors/goggles, bracelets, wristbands, necklaces, etc. For the sake of clarity and simplicity, the embodiments outlined in this specification are exemplified with, and related to, mobile phones, tablets, headphones and head and neck wearables only.

The term collar is herein used as a wearable electronic communication accessory communicatively connected to another communication device, such as a hearing device and which is worn around the neck of a user. The hearing device may be both in-ear versions or sit on the ear with any kind of holder to hold it in place.

The terms wearable and wearable device are used interchangeably and is referring to a wireless device which is worn somewhere on the body of a user. Examples of wearables are watches, wristbands, headsets, headbands, necklaces, necklace-headsets etc.

The term short distance communication refers to any communication with a range limited to about 10 meters. Examples of short distance communication are Body coupled Communication, Wi-Fi, Infrared, ZigBee, Bluetooth and Bluetooth Low Energy.

It should be noted that within this document, the term Bluetooth is used for both Bluetooth and Bluetooth Low Energy, BLE. At present it is not possible to use BLE to send audio signals but the plan is to include this in the 2016 release of BLE.

Body Coupled Communications, BCC, also referred to as Body Based Communication, BBC, or Near Body Communication, NBC, has been proposed as a promising alternative to radio frequency, RF, communication as a basis for Body/Personal Area Network, BAN/PAN, communication. A Body Area Network, BAN, or in other words a Personal Area Network, PAN, is a network with at least two devices communicating through a human body. In other words, BCC allows for an exchange of information between a plurality of devices which are in contact with or in close proximity of a living body. This can be achieved by the transmitting BCC-/BAN-antenna that provide a capacitive or galvanic coupling of low-energy electrical fields onto the body surface, i.e. leakage currents with a small magnitude is set to spread out over the human body. The small currents are then detected by a receiver BCC-/BAN-antenna, located on the same body. Thus, signals are conveyed over the body instead of through the air. As such, the communication is confined to the volume close to the body in contrast to RF communications, where a much larger volume is covered. Therefore, communication is possible between devices situated on, connected to, or placed close to the body. The power consumption of BCC-/BAN-antennas is very low which means that the BCC antennas can be turned on the entire time.

BCC is no new technique, and it has previously been employed within the fields of e.g. medical sensor devices and exercise sensors. Within these fields, sensors attached to the users skin or worn in the close proximity of the body is employed for monitoring different body functions in order to keep track of medical status or fitness parameters of the user. To send the collected data to a central unit e.g. BCC/BBC can be utilized.

As discussed in the summary, the disclosure provides for a device, system and method for monitoring the heart activity of a user by detecting the footprint of the heart. A wireless communication accessory, such as a Bluetooth Stereo Headset, including a collar that is attached around the owner's neck, may be used as carrier for a heart footprint detecting unit, i.e. conductive surfaces or electrodes. It has been found that the electrodes gives a very firm and stable signal regardless of the mechanical and electrical contact challenges related to e.g. physical activity performed by the user, such as dancing or runing.

The pressure effect of the collar is used to secure galvanic contact with the skin, without the need of contact compound e.g. the collar is using its own weight, the gravitation force, to push itself to make galvanic skin contact. The natural elastic pressure, spring pressure, of the collar towards the skin will also enable a solid contact even during exercising and effectively eliminates parasitic signals due to bad contact.

In other words, the accessory uses galvanic contact to detect the electrical footprint of the heart of the user and Bluetooth or BCC to connect with an electronic device, such as a smartphone, and send the detected result to the electronic device for further processing.

Further advantages of using conductive surfaces or electrodes mounted on an existing collar are the enhanced stability and its low production price.

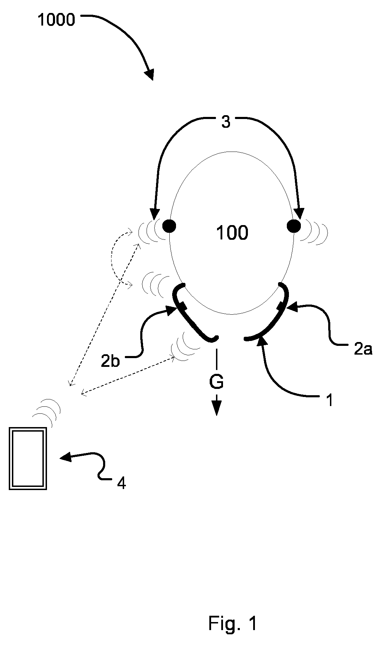

Referring in detail to the drawings, and initially to figure 1, a wearable communication accessory 1, e.g. a collar, in accordance with an embodiment of the present invention is illustrated generally at 1. A wearable communication accessory 1 in this application refers to a wireless communication device configured to be worn by a person. The wearable communication accessory 1 will be referred to below as an accessory 1.

A wireless communication device in accordance with an embodiment of the present invention is illustrated generally at 4. Embodiments of the present invention will be exemplified using a wireless communication device 4 such as a mobile communication device such as a mobile phone. However, it should be appreciated that the invention is as such equally applicable to wireless communication device which have touch detection capabilities. Examples of such devices may for instance be any type of mobile phone, smartphone, laptop (such as standard, ultra-portables, netbooks, and micro laptops) handheld computers, portable digital assistants, tablet computers, touch pads, gaming devices, etc. For the sake of clarity and simplicity, the embodiments outlined in this specification are exemplified with, and related to, mobile phones, tablets, headphones and head and neck wearables only.

In one embodiment a user 100 is wearing and using an accessory 1 connected to an electronic device 4. The accessory may be connected to a hearing device 3 in the form of a pair of earbuds that are wirelessly connected to each other, but they may also be connected to each other via a wire that is worn on the user around the neck. The wearable communication accessory 1 is shown as a collar. In one case the earbuds are wirelessly connected to the collar 1 which is wirelessly connected to the electronic device 4, i.e. a mobile phone. In one case, the hearing device 3 may be wirelessly connected with the collar 1 and wireless connected with the mobile phone 4. The collar comprises electronic modules 5', 5" comprising all electronics of the collar and which also are used as a weight of the collar to further enhance the pressure effect of the construction of the collar. The collar also comprises at least two conductive surfaces or electrodes 2a, 2b, 2c, 2d, 2e positioned at the surface of the housing of the collar at the part that is in direct contact with the skin of the user. In some aspects the conductive contact surfaces 2a, 2b, 2c, 2d, 2e are made of any of silver, plated brass or copper.

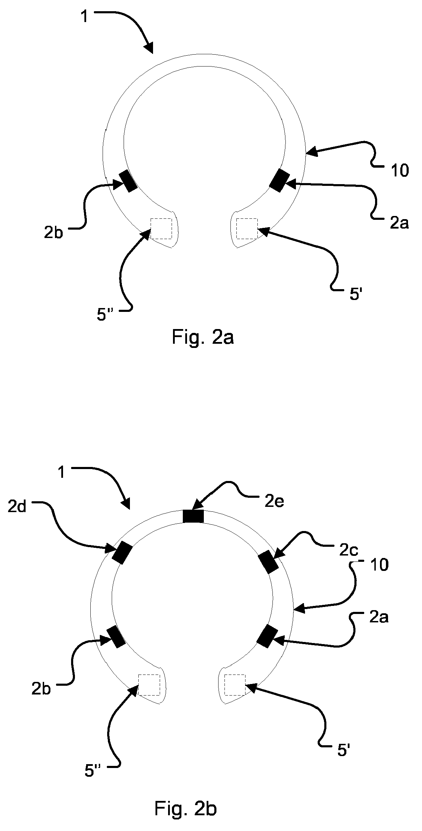

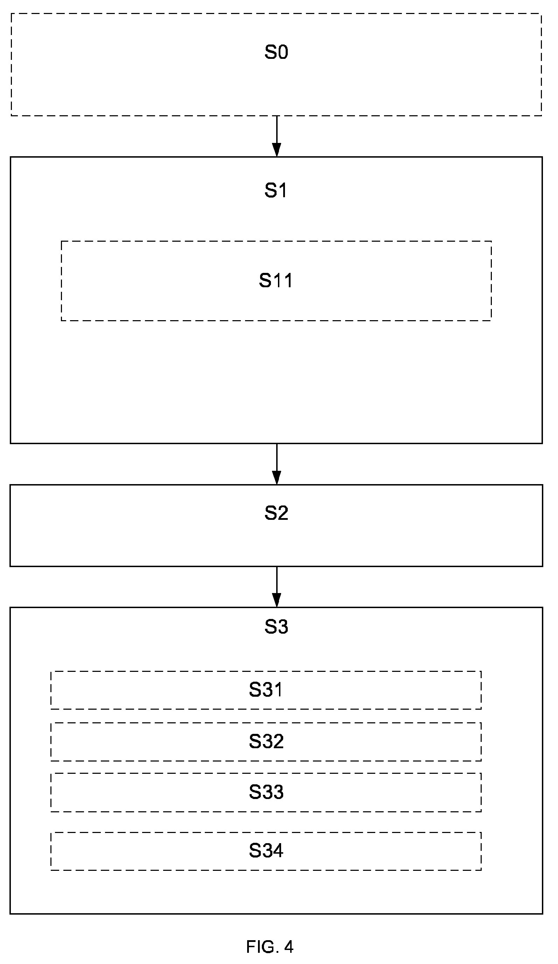

The method will now be described starting from figure 4 depicting example operations which may be taken by the accessory 1 of figure 2a and figure 2b. Figure 4 comprise some operations which are illustrated with a solid border and some operations which are illustrated with a dashed border. It should be appreciated that the operations need not be performed in order. Furthermore, it should be appreciated that not all of the operations need to be performed.

According to some aspect, a method for monitoring an electrical footprint of the heart of a user 100 by using a wireless communication accessory 1 connected to a wireless communication device 4. In one embodiment a wireless communication accessory 1 comprises a housing 10 constructed to be worn around the neck of a user 100. The housing may be formed in the shape of a collar. The accessory further comprises an electronic module 5', 5" comprising a communication circuit 12 and a processor 11. A power supply, not shown may also be provided in the electronic module. The electronic module may be divided into two electronic modules positioned at places of the accessory and connected to each other, wireless or wired. The communication circuit 12, processor 11 and the two or more conductive contact surfaces 2a, 2b, 2c, 2d, 2e are communicatory connected to each other. The housing 10 is constructed to be kept in contact with the skin of the user 100 by using the gravitation force G. The two or more conductive contact surfaces 2a, 2b, 2c, 2d, 2e are positioned on the housing 10 such that the conductive contact surfaces 2a, 2b, 2c, 2d, 2e are in direct contact with the skin of a user 100 when the housing is positioned around the neck of said user 100. In one aspect the housing 10 is constructed to be kept in contact with the skin of the user 100 by using a spring effect of the construction of the housing 10. In one aspect the conductive contact surfaces 2a, 2b, 2c, 2d, 2e are kept in contact with the skin of the user 100 by using the spring effect of the construction of the housing and the gravitation force G acting on the housing for creating a pressure effect of the housing to secure contact with the skin of the user 100.

In a first initial step a connection is established S0 between the accessory 1 and an electronic device 4. The processing circuitry 11 of the accessory 21 and the processing circuitry 21 of the electronic device 4 are configured to establish the connection, using the communication circuitry 12 of the accessory 1, between the accessory 1 and the electronic device 4. According to some aspects the processing circuitry 11, 21 comprises an establisher 111, 211 for establishing the connection. The connection is ether a wireless or a wired connection.

According to some aspects, where the connection between the wearable communication device 1 and an electronic device 4 is wireless, the connection comprises that the connection is a Bluetooth connection or BCC connection. Thus, the functionality is increased when the accessory is wireless accessory. In other words, the accessory is equipped with Bluetooth and/or BCC technology.

In the first step an electrical footprint of the heart of the user is detected S1 by using two or more conductive contact surfaces 2a, 2b, 2c, 2d, 2e positioned on the wireless communication accessory 1. The conductive contact surfaces 2a, 2b, 2c, 2d, 2e may be electrodes or sensors. According to some aspect the wireless communication accessory 1 is provided with four or more conductive contact surfaces 2a, 2b, 2c, 2d, 2e and the detecting comprises selecting S11 a pair of conductive contact surfaces that detects the strongest electrical footprint signal of the heart. The processor 11 is configured to select which pair of conductive contact surfaces detects the strongest electrical footprint signal of the heart, when the accessory is provided with four or more conductive contact surfaces 2a, 2b, 2c, 2d, 2e.

In the second step the detected signal from the wireless communication accessory 1 is transmitted S2 to the wireless communication device 4. The detected signal comprises information defining the electrical footprint of the heart of the user of the accessory 1. The communication circuitry 12 is configured to transmit the detected electrical footprint of the heart of a user 100 to a wireless communication device 4 by using the communication circuitry 12. The processing circuitry 11 is configured to send the signal, using the communication circuitry 12, from the accessory 1 to the electronic device 4. According to some aspects the processing circuitry 11 comprises a sender 112 for sending the signal.

In the third step the transmitted electrical footprint is processed S3. In some aspect the electrical footprint of the heart is an electrocardiogram, ECG, signal and the processor 11 is configured to calculate heart rate beats per minute, bpm, based on the detected electrocardiogram, ECG, signal. According to some aspect processing further comprises amplifying S31 the detected electrocardiogram, ECG, signal with amplitude of 1 mV, subtracting S32 common mode frequency component, the 50/60 Hz frequency component, and filtering S33 out frequency components above 5 Hz and calculating S34 heart rate beats per minute, bpm, based on the detected ECG signal. The processor 11 is configured to amplify the detected ECG signal with amplitude of 1 mV, to subtract common mode frequency component, 50/60 HZ frequency components, and to filter out frequency components above 5 Hz, before calculate the heart rate beats per minute, bpm.

According to some aspects the disclosure provides for a wireless system 1000 for monitoring an electrical footprint of the heart of a user 100. The system comprises a wireless communication device 4, e.g. a mobile phone, a wireless communication accessory 1, e.g. an accessory 4 comprising a collar 10 and a hearing device 3, connected to the wireless communication device 4, The accessory 1 comprising a housing 10 constructed to be worn around the neck of a user 100, a communication circuit 12, two or more conductive contact surfaces 2a, 2b, 2c, 2d, 2e configured to detect the electrical footprint of the heart of a user 100, a processor 11 configured to process the detected electrical footprint. The communication circuit 12, processor 11 and the two or more conductive contact surfaces 2a, 2b, 2c, 2d, 2e are communicatory connected to each other The housing 10 is constructed to be kept in contact with the skin of the user by using the gravitation force, G. The two or more conductive contact surfaces 2a, 2b, 2c, 2d, 2e are positioned on the housing such that the conductive contact surfaces 2a, 2b, 2c, 2d, 2e are in direct contact with the skin of a user 10 when the housing is positioned around the neck of the user 100.

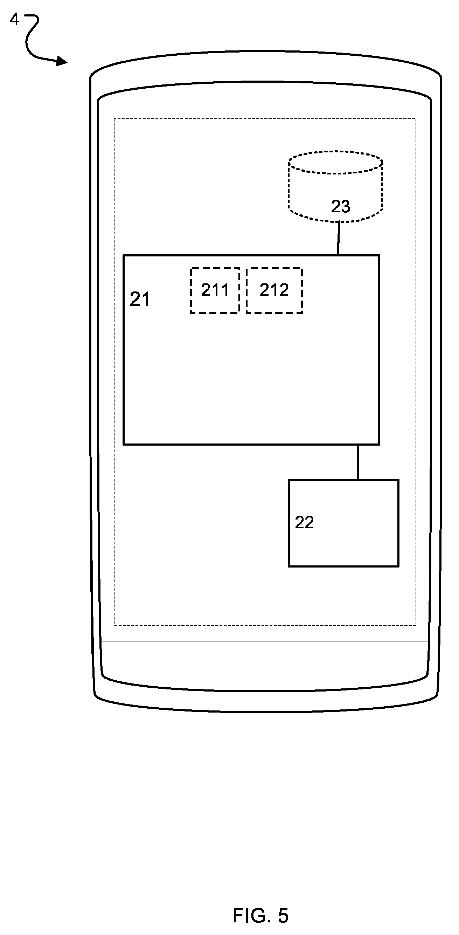

According to some aspects the disclosure provides for a method performed in an electronic device 4, for monitoring the footprint of the heart of the user. As can be seen in figure 5, the electronic device comprises communication circuitry 22 for short distance communication and processing circuitry 21.

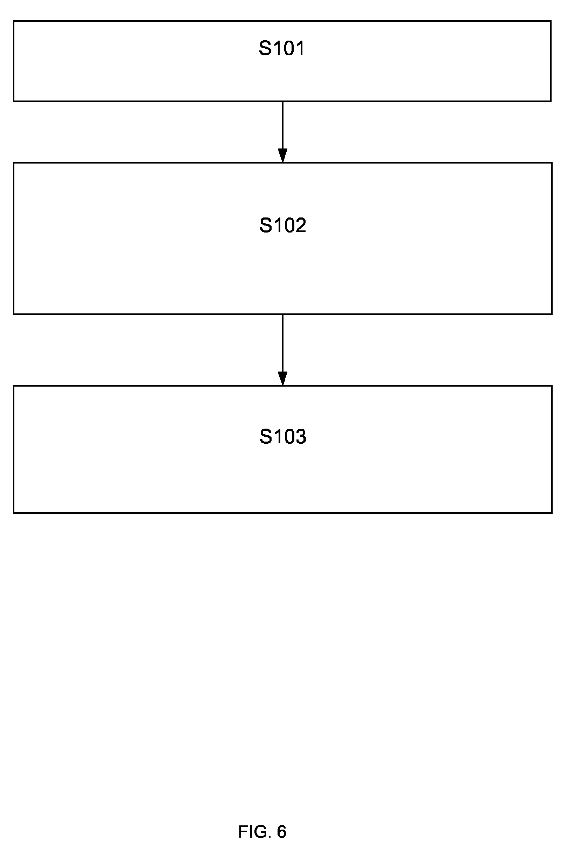

Figure 6 is a flow diagram depicting example operations which may be taken by the electronic device 4 of figure 5. Figure 6 comprise some operations which are illustrated with a solid border and some operations which are illustrated with a dashed border. It should be appreciated that the operations need not be performed in order. Furthermore, it should be appreciated that not all of the operations need to be performed.

According to some aspects, the method of the electronic device 4 comprises the steps of establishing S101 a connection between the electronic device 4 and the accessory 1. The processing circuitry 21 is configured to establish S101 the connection, using the communication circuitry 22, between the electronic device 4 and the accessory 1. According to some aspects, the processing circuitry 21 comprises an establisher 211 for establishing the connection.

According to some aspects, the step of establishing S101 a connection comprises establishing a connection via Bluetooth or Body Coupled Communication, BCC. The functionality is increased when the accessory 4 is a wireless connected accessory. In other words, the electronic device 4 is equipped with Bluetooth and/or BCC technology.

According to some aspects, the method of the electronic device 4 comprises the step of receiving S102, a signal from the accessory 1, the signal comprising information defining whether the accessory has detected an electrical footprint of the heart of the user. The processing circuitry 21 is configured to receive S102 the signal from the accessory 1 using the communication circuitry 22. According to some aspects the processing circuitry comprises a receiver 212 for receiving the signal. The electronic device uses the information to further process S103 the received information.

In some implementations and according to some aspects of the disclosure, the functions or steps noted in the blocks can occur out of the order noted in the operational illustrations. For example, two blocks shown in succession can in fact be executed substantially concurrently or the blocks can sometimes be executed in the reverse order, depending upon the functionality/acts involved. Also, the functions or steps noted in the blocks can according to some aspects of the disclosure be executed continuously in a loop.

The description of the example embodiments provided herein have been presented for purposes of illustration. The description is not intended to be exhaustive or to limit example embodiments to the precise form disclosed, and modifications and variations are possible in light of the above teachings or may be acquired from practice of various alternatives to the provided embodiments. The examples discussed herein were chosen and described in order to explain the principles and the nature of various example embodiments and its practical application to enable one skilled in the art to utilize the example embodiments in various manners and with various modifications as are suited to the particular use contemplated. The features of the embodiments described herein may be combined in all possible combinations of methods, apparatus, modules, systems, and computer program products. It should be appreciated that the example embodiments presented herein may be practiced in any combination with each other.

It should be noted that the word "comprising" does not necessarily exclude the presence of other elements or steps than those listed and the words "a" or "an" preceding an element do not exclude the presence of a plurality of such elements. It should further be noted that any reference signs do not limit the scope of the claims, that the example embodiments may be implemented at least in part by means of both hardware and software, and that several "means", "units" or "devices" may be represented by the same item of hardware.

The various example embodiments described herein are described in the general context of method steps or processes, which may be implemented according to some aspects by a computer program, comprising computer readable code which, when run on an electronic device, causes the electronic device to perform the method according to above. The computer program, embodied in a computer-readable medium, includes computer-executable instructions, such as program code, executed by computers in networked environments. A computer-readable medium may include removable and non-removable storage devices 23 including, but not limited to, Read Only Memory, ROM, Random Access Memory, RAM, compact discs, CDs, digital versatile discs, DVD, etc. Generally, program modules may include routines, programs, objects, components, data structures, etc. that performs particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

In the drawings and specification, there have been disclosed exemplary embodiments. However, many variations and modifications can be made to these embodiments. Accordingly, although specific terms are employed, they are used in a generic and descriptive sense only and not for purposes of limitation, the scope of the embodiments being defined by the following claims.

## Claims

1. An wireless communication accessory (1) comprising:
- a housing (10) constructed to be worn around the neck of a user (100);
- two or more conductive contact surfaces (2a, 2b, 2c, 2d, 2e) configured to detect an electrical footprint of the heart of a user (100);
- an electronic module (5', 5") comprising:
o a communication circuit (12); and
o a processor (11) configured to process said detected electrical footprint, wherein:
- said communication circuit (12), processor (11) and said two or more conductive contact surfaces (2a, 2b, 2c, 2d, 2e) are communicatory connected to each other;
- said housing (10) is constructed to be kept in contact with the skin of the user (100) by using the gravitation force (G); and
- said two or more conductive contact surfaces (2a, 2b, 2c, 2d, 2e) are positioned on said housing (10) such that said conductive contact surfaces (2a, 2b, 2c, 2d, 2e) are in direct contact with the skin of a user (100) when said housing is positioned around the neck of said user (100).

2. The wireless communication accessory (1) according to claim 1, wherein said housing (10) is constructed to be kept in contact with the skin of the user (100) by using a spring effect of the construction of the housing (10).

3. The wireless communication accessory (1) according to claim 1, wherein said accessory is provided with four or more conductive contact surfaces (2a, 2b, 2c, 2d, 2e) and said processor (11) is configured to select which pair of conductive contact surfaces detects the strongest electrical footprint signal of the heart.

4. The wireless communication accessory (1) according to any of the claims 1 to 3, wherein the electrical footprint of the heart is an electrocardiogram (ECG) signal and said processor (11) is configured to:
- calculate heart rate beats per minute, bpm, based on said detected electrocardiogram (ECG) signal.

5. The wireless communication accessory (1) according to claim 4 wherein said processor (11) is configured to:
- amplify said detected electrocardiogram (ECG) signal with amplitude of 1 mV;
- subtract common mode frequency component; and
- filter out frequency components above 5 Hz,
before calculate said heart rate beats per minute, bpm.

6. The wireless communication accessory (1) according to claim 1, wherein said conductive contact surface (2a, 2b, 2c, 2d, 2e) is made of any of silver, plated brass or copper.

7. The wireless communication accessory (1) according to claim 1, wherein the communication circuitry (12) is configured to transmit said detected electrical footprint of the heart of a user (100) to a wireless communication device (4) by using said communication circuitry (12).

8. A wireless system (1000) for monitoring an electrical footprint of the heart of a user (100), wherein said system comprising:
- a wireless communication device (4);
- a wireless communication accessory (1) connected to said wireless communication device (4) and wherein said wireless communication accessory (1) comprising:
o a housing (10) constructed to be worn around the neck of a user (100);
o a communication circuit (12);
o two or more conductive contact surfaces (2a, 2b, 2c, 2d, 2e) configured to detect said electrical footprint of the heart of a user (100); and
o a processor (11) configured to process said detected electrical footprint,
wherein:
o said communication circuit (12), processor (11) and said two or more conductive contact surfaces (2a, 2b, 2c, 2d, 2e) are communicatory connected to each other;
o said housing (10) is constructed to be kept in contact with the skin of the user by using the gravitation force (G); and
o said two or more conductive contact surfaces (2a, 2b, 2c, 2d, 2e) are positioned on said housing such that said conductive contact surfaces (2a, 2b, 2c, 2d, 2e) are in direct contact with the skin of a user (10) when said housing is positioned around the neck of said user (10).

9. The system (1000) according to claim 8, wherein said housing is constructed to be kept in contact with the skin of the user (100) by using a spring effect of the construction of the housing (10).

10. The system (1000) according to claim 8, wherein said accessory (1) is provided with four or more conductive contact surfaces (2a, 2b, 2c, 2d, 2e) and said processor (11, 21) is configured to select which pair of conductive contact surfaces (2a, 2b, 2c, 2d, 2e) detects the strongest electrical footprint signal of the heart.

11. The system (1000) according to claim 8, wherein said processor (11) is configured to:
- amplify said detected electrocardiogram (ECG) signal with amplitude of 1 mV;
- subtract common mode frequency component;
- filter out frequency components above 5 Hz; and
- calculate heart rate beats per minute, bpm, based on said detected electrocardiogram (ECG) signal.

12. A method for monitoring an electrical footprint of the heart of a user (100) by using a wireless communication accessory (1) connected to a wireless communication device (4), wherein the method comprises:
- detecting (S1) said electrical footprint of the heart of the user by using two or more conductive contact surfaces (2a, 2b, 2c, 2d, 2e) positioned on said wireless communication accessory (1);
- transmitting (S2) said detected footprint to said communication device; and
- processing (S3) said transmitted footprint.

13. The method according to claim 12, wherein said wireless communication accessory (1) is provided with four or more conductive contact surfaces and said detecting comprises:
- selecting (S11) a pair of conductive contact surfaces that detects the strongest electrical footprint signal of the heart.

14. The method according to claim 12, wherein said processing further comprises:
- amplifying (S31) said detected electrocardiogram (ECG) signal with amplitude of 1 mV;
- subtracting (S32) common mode frequency component;
- filtering (S33) out frequency components above 5 Hz; and
- calculating (S34) heart rate beats per minute, bpm, based on said detected electrocardiogram (ECG) signal.

15. A computer program, comprising computer readable code which, when run on an electronic device (1, 4), causes the electronic device (1, 4) to perform the method as claimed in any of claims 12-14.
